# EUROPÄISCHE PATENTSCHRIFT

(11) **EP 1 663 116 B1**
(45) Veröffentlichungstag und Bekanntmachung des Hinweises auf die Patenterteilung: **23.06.2010**
(21) Anmeldenummer: 03782476.0
(22) Anmeldetag: 23.12.2003
(51) Int. Cl.: A61K 8/03, A61K 8/37, A61Q 5/06

(54) **3-PHASEN-STYLINGMITTEL**
THREE-PHASE STYLING PRODUCT
AGENT COIFFANT A TROIS PHASES

(30) Priorität: 20.01.2003 DE 10302190
(43) Veröffentlichungstag der Anmeldung: 07.06.2006
(73) Patentinhaber: Henkel AG & Co. KGaA, 40589 Düsseldorf (DE)
(72) Erfinder: EMMERLING, Winfried, 25436 Tornesch (DE); THAMMASIRI, Anja, 22523 Hamburg (DE); BERGEMANN, Uwe, 22459 Hamburg (DE)
(86) Internationale Anmeldenummer: PCT/EP2003/014761
(87) Internationale Veröffentlichungsnummer: WO 2004/064791

(56) Entgegenhaltungen:
- DE-A- 4 103 488
- DE-A- 19 855 767
- FR-A- 2 771 287
- US-A- 5 565 193
- US-A- 5 756 106
- US-A- 6 126 929
- US-A1- 2002 139 384

## Beschreibung

Die Erfindung betrifft stabile, dreiphasige Mittel zur Behandlung von Haaren, enthaltend drei miteinander nicht mischbare Phasen mit einem Gehalt an einer wässrigen Phase, einem wasserunlöslichen Ölkörper und einem flüssigen Ester.

Keratinische Fasern, insbesondere menschliche Haare, werden heutzutage einer Vielzahl von Behandlungen unterzogen. Dabei spielen die Behandlungen, die zu einer permanenten oder temporären Formgebung der Haare dienen, eine wichtige Rolle. Temporäre Formgebungen, die einen guten Halt ergeben sollen, ohne dass das gesunde Aussehen der Haare, wie zum Beispiel deren Glanz, beeinträchtigt wird, können beispielsweise durch Haarsprays, Haarwachse, Fönwellen, Aerosole etc. erzielt werden.

Insbesondere Produkte auf der Basis von Filmbildnern, Weichmachern und synthetischen Ölen finden im Bereich der Formgebung menschlicher Haare vielfältigen Einsatz. Sie bilden beispielsweise im Haarstylingbereich die Grundlage für glanzgebende Haarsprays, wobei der Filmbildner eine Fixierung der Haare und das Öl die Glanzgebung bewirkt. Die Plastifizierung des Filmbildners erfolgt durch den Weichmacher.

Üblicherweise werden Pumphaarsprays und Pumpsprühfestiger als einphasige Produkte in blickdichten Verpackungen angeboten. In der letzten Zeit wurden jedoch verstärkt 2-phasige Produkte in transparenten Verpackungen hergestellt, die dem Verbraucher die Erkennbarkeit des Füllstandes erleichtern und den Produkten eine größere Attraktivität verleihen.

In der EP 1 169 998 A1 werden transparente Aerosolverpackungen mit einem zweiphasigen Mittel offenbart, das aus zwei flüssigen, durch eine scharfe Phasengrenze voneinander getrennten Phasen besteht. Die hydrophile Phase umfasst einen kationischen Haarpflegestoff und die hydrophobe Phase ein verflüssigtes Treibgas.

Aus der DE 197 03 475 A1 sind zweiphasige, sprühfähige Haarpflegemittel bekannt, die in einer hydrophilen Phase kationische oder nichtionische sowie zusätzlich amphotere Tenside und einen Alkohol, und in einer lipophilen Phase eine flüchtige Silikonverbindung und eine nicht-flüchtige Silikonverbindung enthalten.

Zweiphasige Produkte enthalten aber eine Vielzahl unterschiedlicher Wirkstoffe zur Formgebung, Pflege und zum Schutz der Haare, die für den Verbraucher nicht sichtbar sind und bei denen es daher problematisch ist die bessere Wirkung für den Verbraucher glaubhaft zu machen.

Die Ausbildung einer dritten flüssigen Phase durch Zusatz von weiteren kosmetischen Komponenten ist dagegen bisher nicht bekannt, weil diese sich entweder in der einen oder in der anderen Phase lösen oder weil sie Emulgatoren zur Ausbildung einer dreiphasigen Emulsion aus den beiden Phasen sind. Es gab zwar in jüngster Zeit Bestrebungen zur Entwicklung dreiphasiger Systeme mit guter Stabilität für kosmetische Anwendungen, diese Systeme benötigen aber einen hohen Gehalt an Salzen, die bei Anwendungen auf dem Haar aber zu unerwünschten Rückständen führen können.

Ziel war es daher versprühbare Mittel für die Haarbehandlung zu entwickeln, die optisch durch eine Dreiphasenbildung ansprechen und deren kosmetische Wirkung für den Verbraucher noch besser visualisiert werden kann.

In der WO 92/13511 A1 wurden Dreiphasensysteme offenbart, die einen flüssigen, wasserunlöslichen Ölkörper, einen speziellen Emulgator und eine wässrige Phase aufweisen. Diese Dreiphasensysteme sollen sich zur tertiären Ölförderung und für pharmazeutische oder kosmetische Produkte eignen.

Es wurde nun überraschenderweise gefunden, dass durch die Zugabe eines bestimmten Weichmachers zu einem eine wässrige Phase und eine Ölphase ausgebildet werden kann. Die kosmetischen Wirkstoffe können somit zwischen drei Phasen verteilt werden und es ist möglich die Wirksamkeit, gegebenenfalls durch zusätzliche Einfärbung der Phasen, für den Verbraucher besser zu visualisieren.

Durch die bessere Visualisierung wird auch die Glaubwürdigkeit eines komplexen Mittels mit verbesserter Wirkung gesteigert.

Die drei Phasen sind stabil und lassen sich derart vermischen, dass alle drei Komponenten gleichzeitig beim Pumpvorgang zur Anwendung kommen und sich nach der Anwendung wieder in drei Phasen aufteilen.

Gegenstand der Erfindung sind daher stabile, dreiphasige, versprühbare Haarbehandlungsmittel, umfassend drei nicht miteinander mischbare Flüssigkeitsphasen, die aus
(A) einer wässrigen Phase,
(B) einer Ölphase und
(C) einer Phase, enthaltend einen trifunktionalen Ester,
   zusammengesetzt sind,
   - wobei der Ester aus den beiden Einheiten a) und b) zusammengesetzt ist, in denen
      • die Einheit a) für Alkohole und Carbonsäuren mit Kettenlängen von 2 bis 10 C-Atomen steht und
      • die Einheit b) für Verbindungen der Formel (I) steht, 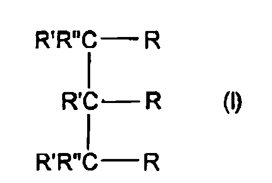 in der die Reste R unabhängig voneinander die Gruppen -OH oder -COOH und R', R" unabhängig voneinander die Gruppen -H, -OH oder -C₁-C₄-Alkyl bedeuten können,
   - wobei der Gehalt an trifunktionalem Ester 10 bis 50 Gew.-%, bezogen auf das Gesamtgewicht des Mittels, beträgt und
   - wobei trifunktionale Ester für solche Ester stehen, bei denen alle drei Reste R der Formel (I) mit Alkoholen und/oder Carbonsäuren mit Kettenlängen von 2 bis 10 C-Atomen verestert wurden.

Bevorzugte Verbindungen der Formel (I) sind solche, bei denen die Reste R für eine Hydroxygruppen stehen, insbesondere bevorzugt ist Glycerin.

Bevorzugte Ester im Sinne der Erfindung sind Ester, die sich aus Glycerin und Carbonsäuren mit 2 bis 6 C-Atomen zusammensetzen.

Ein ganz besonders bevorzugter Ester im Sinne der Erfindung ist Glycerintriacetat.

Weiterhin sind bevorzugte Verbindungen der Formel (I) solche, bei denen die Reste R für -COOH-Gruppen stehen, insbesondere bevorzugt ist Citronensäure.

Bevorzugte Ester im Sinne der Erfindung sind auch solche, die sich aus Citronensäure und Alkoholen mit 2 bis 6 C-Atomen zusammensetzen.

Ein weiterhin ganz besonders bevorzugter Ester im Sinne der Erfindung ist Triethylcitrat.

Erfindungsgemäß ist ein Estergehalt, bezogen auf das Gesamtgewicht des Mittels, von 10 bis 50 Gew.-%.
Die zweite zwingende Komponente der Erfindung ist die Ölkomponente, die bei der Haarbehandlung für die Erzeugung von Glanz auf dem Haar verantwortlich ist.

Die Ölkomponente kann prinzipiell sowohl aus pflanzlichen, als auch aus mineralischen oder synthetischen Ölen, sowie aus Gemischen dieser Komponenten, ausgewählt sein.

Als natürliche (pflanzliche) Öle werden üblicherweise Triglyceride und Mischungen von Triglyceriden eingesetzt. Bevorzugte natürliche Öle im Sinne der Erfindung sind Kokosnussöl, (süßes) Mandelöl, Walnussöl, Pfirsichkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokemöl, Wiesenschaumkrautöl, Distelöl, Macadamianussöl, Traubenkemöl, Aprikosenkemöl, Olivenöl, Weizenkeimöl, Kürbiskernöl, Malvenöl, Haselnussöl, Safloröl, Canolaöl, Sasanquaöl, Jojobaöl und Shea-Butter.

Erfindungsgemäß besonders bevorzugt sind (süßes) Mandelöl, Avocadoöl, Sojaöl, Sesamöl, Sonnenblumenöl, Palmkernöl, Mangokernöl, Macadamianussöl, Aprikosenkernöl, Olivenöl, Weizenkeimöl, Kürbiskernöl, Malvenöl, Haselnussöl und Jojobaöl.

Als mineralische Öle kommen insbesondere Mineralöle, Paraffin- und Isoparaffinöle sowie synthetische Kohlenwasserstoffe zum Einsatz. Ein erfindungsgemäß einsetzbarer Kohlenwasserstoff ist beilspielsweise das als Handelsprodukt erhältliche 1,3-Di-(2-ethylhexyl)-cyclohexan (Cetiol^{®} S).

Erfindungsgemäß bevorzugte mineralische Öle sind Paraffinöle.

Als synthetische Öle kommen Silikonverbindungen, insbesondere Dialkyl- und Alkylarylsilikone, wie beispielsweise Dimethylpolysiloxan und Methylphenylpolysiloxan, sowie deren hydroxy-terminierte, alkoxylierte und quaternierte Analoga in Betracht. Beispiele für solche Silikone sind die von Dow Corning unter den Bezeichnungen DC 190, DC 200, DC 344 und DC 345 (Cyclomethicone) vertriebenen Produkte.

Erfindungsgemäß einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Erfindungsgemäß besonders bevorzugt ist der Di-n-octylether, der im Handel unter der Bezeichnung Ceitiol^{®} OE erhältlich ist.

Wenngleich in der Regel bei Raumtemperatur, d.h. bei 25°C, flüssige Öle eingesetzt werden, so umfasst die Erfindung jedoch auch die Verwendung von Mischungen von flüssigen und festen Ölkomponenten, sofern diese Mischungen bei Raumtemperatur flüssig sind.

In einer weiteren bevorzugten Ausführungsform der Erfindung umfasst die Phase (B) anstatt der Ölkomponente flüssige, halogenierte Kohlenwasserstoffe als dritte Phase.
Diese können ausgewählt sein aus handelsüblichen chlorierten und/oder fluorierten Kohlenwasserstoffen. Insbesondere geeignet im Sinne der Erfindung sind Ethyl-perfluorbutylether und/oder Perfluordecahydronaphthalin.

Die Ölkomponente oder die Mischung der Ölkomponenten oder die flüssigen halogenierten Kohlenwasserstoffe sind in den erfindungsgemäßen Mitteln vorzugsweise in Mengen von 2 bis 50 Gew.-%, bezogen auf das Gesamtgewicht des Mittels, enthalten.

Besonders bevorzugt sind Mengen der Ölkomponente(n) im Bereich von 10 bis 40 Gew.-%, bezogen auf das Gesamtgewicht des Mittels.

Die wässrige Phase umfasst erfindungsgemäß einen Mengenanteil im Bereich von 2 bis 50 Gew.%, bezogen auf das Gesamtgewicht des Mittels.

Das Gesamtsystem kann auch Alkohole wie Ethanol oder Isopropanol enthalten. Der Alkoholgehalt umfasst erfindungsgemäß 0 - 30 Gew.-%, bevorzugt 0 bis 20 Gew.-% und insbesondere 0 bis 10 Gew.-%, jeweils bezogen auf das Gesamtgewicht des Mittels.

In einer weiteren bevorzugten Ausführungsform der Erfindung enthalten die Mittel weiterhin mindestens einen wasserlöslichen Filmbildner, bei dem es sich üblicherweise um ein Polymer handelt.

Bevorzugte wasserlösliche Polymere können nichtionogener, amphoterer, zwitterionischer oder anionischer Natur sein.

Als nichtionogene Polymere kommen erfindungsgemäß beispielsweise die folgenden Verbindungen in Frage:
- Polyvinylpyrrolidone, wie sie beispielsweise unter der Bezeichnung Luviskol^{®} von der Firma BASF vertrieben werden, beispielsweise Luviskol^{®} K 30,
- Vinylpyrrolidon/Vinylacetat-Copolymere, wie sie beispielsweise unter dem Warenzeichen Luviskol^{®} von der Firma BASF vertrieben werden. Luviskol^{®} VA 64 und Luviskol^{®} VA 73 vertrieben werden,
- Celluloseether, wie Hydroxypropylcellulose, Hydroxytehylcellulose und Methylhydroxypropylcellulose, wie sie beispielsweise unter dem Warennamen Cumlina^{®} und Benecel^{®} von der Firma Aqualon vertrieben werden.
Besonders geeignete wasserlösliche, nichtionogene Polymere im Sinne der Erfindung sind die unter den Handelsnamen Luviskvl^{®} K 30, Luviskol^{®} VA 64 und Luviskol^{®} VA 73 vertriebenen Produkte.

Besonders geeignete wasserlösliche, nichtionogene Polymere im Sinne der Erfindung sind die unter den Handelsnamen Luviskol^{®} K 30, Luviskol^{®} VA 64 und Luviskol^{®} VA 73 vertriebenen Produkte.

Erfindungsgemäß geeignete amphotere Polymere sind beispielsweise die unter den Bezeichnungen Amphomer^{®} und Amphomer^{®} LV-71 von der Firma Delft National vertriebenen Octylacrylamid/Methylmethacrylat/tert.-Butylaminoethylmethacrylat/2-Hydroxypropylmethacrylat-Copolymere.

Erfindungsgemäß geeignete anionische Polymere sind u.a.
- Vinylacetat/Crotonsäure-Copolymere, wie sie beispielsweise unter den Bezeichnungen Resyn^{®} von der Firma National Starch, Luviset^{®} von der Firma BASF und Gafset^{®} von der Firma GAF im Handel sind,
- Vinylpyrrolidon/Vinylacrylat-Copolymere, erhältlich beispielsweise unterdem Warenzeichen Luviflex^{®} der Firma BASF. Ein bevorzugtes Beispiel für diese Produkte isr das unter dem Warenzeichen Luviflex^{®} VBM-35 erhältliche Vinylpyrrolidon/Acrylat-Terpolymere,
- Acrylsäure/Ethylacrylat/N-tert.-Butylarcylamid-Terpolymere, die beispielsweise unter der Bezeichnung Ultrahold^{®} strong von der Firma BASF vertrieben werden.

Die wasserlöslichen Filmbildner können in den erfindungsgemäßen Mitteln in Mengen von 0,5 bis 15 Gew.-%, insbesondere 2 bis 8 Gew.-%, bezogen auf das Gesamtgewicht der Mittel, eingesetzt werden.

In einer weiteren bevorzugten Ausführungsform der Erfindung enthalten die Mittel weiterhin öl- und wasserlösliche Haarpflegestoffe, öl- und wasserlösliche UV-Absorber, Parfümöle und/oder Farbstoffe.

Unter öl- und wasserlöslichen Haarpflegestoffen werden erfindungsgemäß kationische oder kationaktive haarpflegende Stoffe, wie kationische Polymere, kationische Tenside oder kationisch derivatisierte Proteinhydrolysate, Vitamine und Vitaminderivate sowie öllösliche Wachse verstanden.

Beispiele für erfindungsgemäß geeignete kationische Polymere sind kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationi-sche Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR-A 2252840 sowie deren vernetzte wasserlöslichen Polymere, kationische Chitinderivate, wie beispiels-weise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Die kationischen Polymere werden in den erfindungsgemäßen Zusammensetzungen bevorzugt in einer Menge von 0,1 bis 10 Gew.-%, insbesondere in einer Menge von 0,2 bis 2 Gew.-%, jeweils bezogen auf das Gesamtgewicht der Zusammensetzungen, eingesetzt.

Als kationische Tenside können erfindungsgemäß bevorzugt Tenside vom Typ der quartären Ammoniumverbindungen, der Esterquats und der Amidoamine eingesetzt werden. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quatemierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Die kationischen Tenside sind in den erfindungsgemäß verwendeten Mitteln bevorzugt in Mengen von 0,05 bis 2 Gew.-%, bezogen auf das gesamte Mittel, enthalten. Mengen von 0,1 bis 1 Gew.-% sind besonders bevorzugt.

In einer weiteren Ausführungsform der erfindungsgemäßen Mittel kann die Wirkung durch die Verwendung von Proteinhydrolysaten und deren kationischen Derivaten weiter gesteigert werden. Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Gluadin^{®} (Cognis), Lexein^{®} (Inolex), Crolastin^{®} (Croda) oder Crotein^{®} (Croda) vertrieben.

Die Proteinhydrolysate werden erfindungsgemäß bevorzugt in einer Menge von 0,1 bis 6 Gew.-%, insbesondere in einer Menge von 0,2 bis 2 Gew.-%, jeweils bezogen auf das Gesamtgewicht der Mittel, eingesetzt.

Unter erfindungsgemäß bevorzugten Vitaminen und Vitaminderivaten werden solche Vitamine, Pro-Vitamine und Vitaminvorstufen bevorzugt, die üblicherweise den Gruppen A, B, C, E, F und H zugeordnet werden.

Zur Gruppe der als **Vitamin A** bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Zubereitungen enthalten die Vitamin A-Komponente bevorzugt in Mengen von 0,05-1 Gew.-%, bezogen auf die gesamte Zubereitung.

Zur **Vitamin B-Gruppe** oder Vitamin B-Komplex gehören u. a.
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mittel bevorzugt in Mengen von 0,05 bis 1 Gew.-%, bezogen auf das gesamte Mittel, enthalten ist.
Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B5-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

**Vitamin C** (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters kann bevorzugt sein. Die Verwendung in Kombination mit Tocopherolen kann ebenfalls bevorzugt sein.

**Vitamin E** (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

**Vitamin F.** Unter dem Begriff "Vitamin F" werden üblicherweise essentielle Fettsäuren, insbesondere Linolsäure, Linolensäure und Arachidonsäure, verstanden.

**Vitamin H.** Als Vitamin H wird die Verbindung (3aS,4S, 6aR)-2-Oxohexahydrothienol[3,4-*d*]-imidazol-4-valeriansäure bezeichnet, für die sich aber zwischenzeitlich der Trivialname Biotin durchgesetzt hat. Biotin ist in den erfindungsgemäßen Mitteln bevorzugt in Mengen von 0,0001 bis 0,1 Gew.-%, insbesondere in Mengen von 0,001 bis 0,01 Gew.-% enthalten.

Bevorzugt enthalten die erfindungsgemäßen Mittel Vitamine, Provitamine und Vitaminvorstufen aus den Gruppen A, B, E und H.

Panthenol und seine Derivate sowie Nicotinsäureamid und Biotin sind besonders bevorzugte pflegende Wirkstoffe, insbesondere bevorzugt ist Panthenol und seine Derivate.

UV-Filter im Sinne der Erfindung sind ausgewählt aus substituierten Benzophenonen, p-Aminobenzoesäureestem, Diphenylacrylsäureestem, Zimtsäureestern, Salicylsäureestern, Benzimidazolen und o-Aminobenzoesäureestem.

Insbesondere bevorzugte UV-Filter sind erfindungsgemäß 4-Aminobenzoesäure, N,N,N-Trimethyl-4-(2-oxoborn-3-ylidenmethyl)anilin-methylsulfat, 3,3,5-Trimethylcyclohexylsalicylat (Homosalate), 2-Hydroxy-4-methoxy-benzophenon (Benzophenone-3; Uvinul^{®}M40, Uvasorb^{®}MET, Neoheliopan^{®}BB, Eusolex^{®}4360), 1-(4-tert.-Butylphenyl)-3-(4-methoxyphenyl)-propan-1,3-dion (Butyl methoxydibenzoylmethane; Parsol^{®}1789, Eusolex^{®}9020), ethoxylierte 4-Aminobenzoesäureethylester (PEG-25 PABA; Uvinul^{®}P25), 4-Dimethylaminobenzoesäure-2-ethylhexylester (Octyl Dimethyl PABA; Uvasorb^{®}DMO, Escalol^{®}505, Eusolex^{®}6007), Salicylsäure-2-ethylhexylester (Octyl Salicylat; Escalol^{®}587, Neoheliopan^{®}OS, Uvinul^{®}O18), 4-Methoxyzimtsäure-isopentylester (Isoamyl p-Methoxycinnamate; Neoheliopan^{®}E 1000), 4-Methoxyzimtsäure-2-ethylhexylester (Octyl Methoxycinnamate; Parsol^{®}MCX, Escalol^{®}557, Neoheliopan^{®}AV), 3-(4'-Methylbenzyliden)-D,L-Campher (4-Methylbenzylidene camphor; Parsol^{®}5000, Eusolex^{®}6300), 3-Benzyliden-campher (3-Benzyllidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäureethylester, Polymere des N-{(2-und 4-)[2-oxoborn-3-ylidenmethyl]benzyl}-acrylamid, 2,4-Dihydroxybenzophenon (Benzophenone-1; Uvasorb^{®}20H, Uvinul^{®}400), 1,1'-Diphenylacrylonitrilsäure-2-ethylhexyl-ester (Octostyrene; Eusolex^{®}OCR, Neoheliopan^{®}Type 303, Uvinul^{®}N 539 SG), o-Aminobenzoesäure-menthylester (Menthyl Anthranilate; Neoheliopan^{®}MA), 2,2',4,4'-Tetrahydroxybenzophenon (Benzophenone-2; Uvinul^{®}D50), 2,2'-Dihydroxy-4,4'-dimethoxy-benzophenon (Benzophenone-6) und 2-Cyano-3,3'-diphenylacrylsäure-2'-ethylhexylester.

Erfindungsgemäß besonders bevorzugt ist Benzophenon-3.

Ein Beispiel für einen erfindungsgemäß insbesondere bevorzugten, wasserlöslichen UV-Filter ist 2-Hydroxy-4-methoxybenzophenon-5-sulfonsäure (Uvasorb^{®} S 5, Uvinul^{®} MS 40, Escalol^{®} 577; Benzophenone-4).

Die UV-Filter werden erfindungsgemäß in Mengen von 0,01 bis 30 Gew.-%, insbesondere in Mengen von 0,05 bis 5 Gew.-%, jeweils bezogen auf das Gesamtgewicht des Mittels, eingesetzt.

Als Farbpigmente eignen sich beispielsweise die Pigmente mit den C.I.-Namen Pigment Red 57:1, Pigment Red 57:2, Pigment Red 172, Pigment Red 90:1, Pigment Yellow 100, Pigment Yellow 115, Pigment Red 174, Pigment Red 4, Pigment Blue 29, Pigment Violet 15, Pigment Violet 16, Pigment Red 29, Pigment Green 17, Pigment Green 18, Natural Red 4, Pigment White 6, Pigment White 14 und Pigment White 31.

Als öllösliche Wachse können erfindungsgemäß alle Wachse eingesetzt werden, die einen Schmelzpunkt im Bereich von 40 bis 90°C aufweisen. Als Wachse im Sinne der Erfindung sind alle Stoffe einsetzbar, wie sie z.B. in Ullmanns Enzyklopädie der technischen Chemie, 4. Auflage, Band 24, Seite 3, linke Spalte, aufgeführt sind und physiologisch verträglich sind.

Bevorzugt werden die Wachse aus pflanzlichen, tierischen und mineralischen Wachsen ausgewählt, wobei solche Wachse bevorzugt sein können, die einen Schmelzpunkt im Bereich von 50 bis 85°C, insbesondere von 60 bis 75°C, aufweisen.

Erfindungsgemäß bevorzugte Wachse sind Bienenwachs (Cera Alba), Carnaubawachs, Candelillawachs, Montanwachs, mikrokristalline Wachse (mikrokristalline Paraffine) Cetylpalmitat, oder Gemische dieser Wachse.

Bevorzugte Wachsmischungen im Sinne der Erfindung sind beispielsweise die unter den Bezeichnungen "Spezialwachs 7686 OE" (Mischung aus Cetylpalmitat, Bienenwachs, mikrokristallinem Wachs und Polyethylen mit einem Schmelzbereich von 73 bis 75°C - Hersteller: Kahl & Co.), Polywax^{®} GP 200 (Mischung aus Stearylalkohol und Polyethylenglycolstearat mit einem Schmelzpunkt von 47 bis 51°C - Hersteller: Croda) und "Weichceresin® FL 400" (ein Vaseline/Vaselinöl/Wachs-Gemisch mit einem Schmelzpunkt von 50 bis 54°C - Hersteller: Parafluid Mineralölgesellschaft) im Handel erhältlich sind.

Als Wachskomponente können erfindungsgemäß auch flüssige Wachse, wie beispielsweise Jojobaöl eingesetzt werden.

Bezüglich weiterer üblicher Inhaltsstoffe wird ausdrücklich auf die dem Fachmann bekannten Monographien, beispielsweise K. Schrader, Grundlagen und Rezepturen der Kosmetika, Dr. Alfred Hüthig Verlag, Heidelberg, verwiesen.

Die Konfektionierung der erfindungsgemäßen Mittel unterliegt keinerlei Beschränkungen. Sie können als Lotion, Pumphaarspray, Pumpsprühfestiger, Pflegespray, Anti-Frizz-Spray, Antitanglingspray oder Glanzspray konfektioniert werden.

Ein zweiter Gegenstand der Erfindung ist ein Verfahren zum Styling von Haaren, bei dem das erfindungsgemäße Mittel auf die Haare aufgebracht, die Frisur in Form gebracht und die Haare anschließend mit Heißluft oder an der Luft getrocknet werden.

Ein dritter Gegenstand der Erfindung ist die Verwendung des erfindungsgemäßen Mittels zum Styling von Haaren.

Ein vierter Gegenstand der Erfindung ist die Verwendung des erfindungsgemäßen Mittels zur Erzeugung von Glanz auf Haaren.

Das folgende Beispiel soll den Erfindungsgegenstand näher erläutern, ohne ihn darauf zu beschränken.

### Ausführunasbeispiel:

### Glanzspray:

Die unten stehenden Mengenangaben beziehen sich auf Gew.-%.

In

| | |
|---|---|
| *Wasser* | *49,65,* |

das auf ca. 65°C erwärmet wurde, wird unter Rühren

| | |
|---|---|
| *PVP¹* | *5,00* |

| | |
|---|---|
| ¹ Luviskol^{®}K 30 Pulver (INCI: Polyvinylpyrrolidone; 95-100% AS) | |

zugegeben und klar gelöst.
Danach wurden nacheinander unter Rühren und ohne Wärmezufuhr

| | |
|---|---|
| *Panthenol* | *0,20* |
| *Dimethicone* | *4,00* |
| *Trisiloxane* | *11,00* |
| *Essential Oil* | *0,15* |
| *Triethylcitrat* | *29,00* |
| *Isoamyl-p-methoxycinnamate²* | *1,00* |

| | |
|---|---|
| ² Neoheliopan^{®}E 1000 (INCI: Isoamyl p-Methoxycinnamate) | |

zugegeben.

Es erfolgte die Ausbildung von 3 Phasen, die sich durch kräftiges Schütteln vermischten und sich beim Stehen lassen wieder aufteilten.

## Patentansprüche

1. Stabiles, dreiphasiges, versprühbares Haarbehandlungsmittel, **dadurch gekennzeichnet, dass** es aus drei nicht miteinander mischbaren Flüssigkeitsphasen besteht, die
(A) eine wässrige Phase,
(B) eine Ölphase und
(C) eine Phase, enthaltend einen trifunktionalen Ester, umfassen,
- wobei der Ester aus den beiden Einheiten a) und b) zusammengesetzt ist, in denen
• die Einheit a) für Alkohole oder Carbonsäuren mit Kettenlängen von 2 bis 10 C-Atomen steht und
• die Einheit b) für Verbindungen der Formel (I) steht, 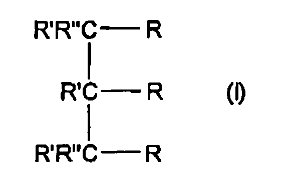 in der die Reste R unabhängig voneinander die Gruppen -OH oder -COOH und R', R" unabhängig voneinander die Gruppen - H, -OH oder C₁-C₄-Alkyl bedeuten können,
- wobei der Gehalt trifunktionalem Ester 10 bis 50 Gew.-%, bezogen auf das Gesamtgewicht des Mittels, beträgt und
- wobei trifunktionale Ester für solche Ester stehen, bei denen alle drei Reste R der Formel (I) mit Alkoholen und/oder Carbonsäuren mit Kettenlängen von 2 bis 10 C-Atomen verestert wurden.

2. Mittel nach Anspruch 1, **dadurch gekennzeichnet, dass** die Reste R für Hydroxygruppen stehen.

3. Mittel nach Anspruch 2, **dadurch gekennzeichnet, dass** die Verbindung der Formel (I) für Glycerin steht.

4. Mittel nach einem der Ansprüche 2 oder 3, **dadurch gekennzeichnet, dass** sich der Ester aus Glycerin und Carbonsäuren mit 2 bis 6 C-Atomen zusammensetzt.

5. Mittel nach einem der Ansprüche 2 bis 4, **dadurch gekennzeichnet, dass** der Ester Glycerintriacetat ist.

6. Mittel nach Anspruch 1, **dadurch gekennzeichnet, dass** die Reste R für -COOH-Gruppen stehen.

7. Mittel nach Anspruch 6, **dadurch gekennzeichnet, dass** die Verbindung nach Formel (I) für Citronensäure steht.

8. Mittel nach einem der Ansprüche 6 oder 7, **dadurch gekennzeichnet, dass** sich der Ester aus Citronensäure und Alkoholen mit 2 bis 6 C-Atomen zusammensetzt.

9. Mittel nach einem der Ansprüche 6 bis 8, **dadurch gekennzeichnet, dass** der Ester Triethylcitrat ist.

10. Mittel nach einem der Ansprüche 1 bis 9, **dadurch gekennzeichnet, dass** die Ölphase ausgewählt ist aus pflanzlichen, mineralischen und synthetischen Ölen, sowie aus Gemischen dieser Komponenten.

11. Mittel nach einem der Ansprüche 1 bis 10, **dadurch gekennzeichnet, dass** anstatt der Ölphase (B) ein flüssiger, halogenierter Kohlenwasserstoff als dritte Phase in dem Mittel enthalten ist.

12. Mittel nach Anspruch 11, **dadurch gekennzeichnet, dass** die flüssigen, halogenierten Kohlenwasserstoffe ausgewählt sind aus Ethyl-perfluorbutylether und/oder Perfluordecahydronaphthalin.

13. Mittel nach einem der Ansprüche 1 bis 12, **dadurch gekennzeichnet, dass** die Phase (B) in einer Menge von 2 bis 50 Gew.-%, bezogen auf das Gesamtgewicht des Mittels, vorliegt.

14. Mittel nach einem der Ansprüche 1 bis 13, **dadurch gekennzeichnet, dass** die Wasserphase in einer Menge von 2 bis 50 Gew.-%, bezogen auf das Gesamtgewicht des Mittels, vorliegt.

15. Mittel nach einem der Ansprüche 1 bis 14, **dadurch gekennzeichnet, dass** weiterhin wasserlösliche Filmbildner enthalten sind.

16. Mittel nach einem der Ansprüche 1 bis 15, **dadurch gekennzeichnet, dass** weiterhin öl- oder wasserlösliche Pflegestoffe, W-Absorber, Parfümöle und/oder Farbstoffe enthalten sind.

17. Mittel nach einem der Ansprüche 1 bis 16, **dadurch gekennzeichnet, dass** sie Panthenol enthalten.

18. Verfahren zum Styling von Haaren, **dadurch gekennzeichnet, dass** ein Mittel nach einem der Ansprüche 1 bis 17 auf die Haare aufgebracht, die Frisur In Form gebracht und die Haare anschließend mit Heißluft oder an der Luft getrocknet werden.

19. Verwendung eines Mittels nach einem der Ansprüche 1 bis 17 zum Styling von Haaren.

20. Verwendung eines Mittels nach einem der Ansprüche 1 bis 17 zur Erzeugung von Glanz auf Haaren.

## Claims

1. A stable, three-phase, sprayable hair treating agent, **characterized in that** it consists of three liquid phases which are not miscible with each other, which comprises
(A) an aqueous phase,
(B) an oil phase, and
(C) a phase containing a trifunctional ester,
- wherein the ester is composed from both units a) and b), in which
• unit a) represents alcohols or carboxylic acids with a chain length from 2 to 10 C atoms and
• unit b) represents compounds of formula (I), 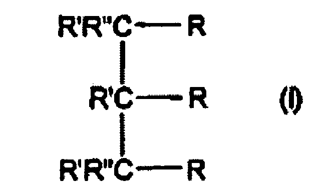 wherein the radicals R may independently of each other stand for the groups -OH or -COOH and R', R" may independently of each other stand for the groups -H, -OH or -(C₁-C₄)-alkyl,
- wherein the content of trifunctional ester is from 10 to 50% by weight based on the total weight of the agent, and
- wherein trifunctional esters represent esters in which all three radicals R of the formula (I) have been esterified with alcohols and/or carboxylic acids with a chain length from 2 to 10 C atoms.

2. The agent according to claim 1, **characterized in that** the radicals R represent hydroxy groups.

3. The agent according to claim 2, **characterized in that** the compound of formula (I) represents glycerol.

4. The agent according to any of claims 2 or 3, **characterized in that** the ester is composed of glycerol and carboxylic acids with 2 to 6 C atoms.

5. The agent according to any of claims 2 to 4, **characterized in that** the ester is glycerol triacetate.

6. The agent according to claim 1, **characterized in that** the radicals R represent -COOH groups.

7. The agent according to claim 6, **characterized in that** the compound according to formula (I) represents citric acid.

8. The agent according to any of claims 6 or 7, **characterized in that** the ester is composed from citric acid and alcohols with 2 to 6 C atoms.

9. The agent according to any of claims 6 to 8, **characterized in that** the ester is triethyl citrate.

10. The agent according to any of claims 1 to 9, **characterized in that** the oil phase is selected from vegetable, mineral and synthetic oils, as well as from mixtures of these components.

11. The agent according to any of claims 1 to 10, **characterized in that** instead of the oil phase (B) a liquid halogenated hydrocarbon is contained as a third phase in the agent.

12. The agent according to claim 11, **characterized in that** the liquid halogenated hydrocarbons are selected from ethyl perfluorobutylether and/or perfluorodecahydronaphthalene.

13. The agent according to any of claims 1 to 12, **characterized in that** the phase (B) exists in an amount from 2 to 50% by weight based on the total weight of the agent.

14. The agent according to any of claims 1 to 13, **characterized in that** the aqueous phase exists in an amount from 2 to 50% by weight, based on the total weight of the product.

15. The agent according to any of claims 1 to 14, **characterized in that** further water-soluble film-forming agents are contained.

16. The agent according to any of claims 1 to 15, **characterized in that** further oil- or water-soluble care substances, UV-absorbents, perfume oils and/or dyes are contained.

17. The agent according to any of claims 1 to 16, **characterized in that** they contain panthenol.

18. A method for styling hair, **characterized in that** an agent according to any of claims 1 to 17 is applied on the hair, the hair style is formed and the hair are then dried with hot air or in the air.

19. The use of an agent according to any of claims 1 to 17 for the styling of hair.

20. The use of an agent according to any of claims 1 to 17 for producing shininess on hair.

## Revendications

1. Agent de traitement capillaire stable, triphasique, atomisable, **caractérisé en ce qu'**il est constitué par trois phases liquides non miscibles l'une avec l'autre, qui comprennent
(A) une phase aqueuse,
(B) une phase huileuse, et
(C) une phase contenant un ester trifonctionnel,
- l'ester étant composé par les deux unités a) et b) dans lesquelles :
• l'unité a) représente des alcools et des acides carboxyliques possédant des longueurs de chaînes de 2 à 10 atomes de carbone ; et
• l'unité b) représente des composés répondant à la formule (I) : 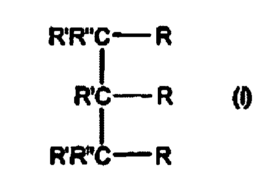 dans laquelle les radicaux R peuvent représenter, indépendamment l'un de l'autre, un groupe -OH ou un groupe -COOH et les radicaux R', R" peuvent représenter, indépendamment les uns des autres, un atome d'hydrogène, un groupe -OH ou un groupe alkyle en C₁-C₄,
- la teneur en ester trifonctionnel s'élevant de 10 à 50 % en poids, rapportés au poids total de l'agent ; et
- des esters trifonctionnels représentant des esters dans lesquels l'ensemble des trois radicaux R répondant à la formule (I) ont été estérifiés avec des alcools et/ou avec des acides carboxyliques possédant des longueurs de chaînes de 2 à 10 atomes de carbone.

2. Agent selon la revendication 1, **caractérisé en ce que** les radicaux R représentent des groupes hydroxyle.

3. Agent selon la revendication 2, **caractérisé en ce que** le composé répondant à la formule (I) représente du glycérol.

4. Agent selon l'une quelconque des revendications 2 ou 3, **caractérisé en ce que** l'ester se compose de glycérol et d'acides carboxyliques contenant de 2 à 6 atomes de carbone.

5. Agent selon l'une quelconque des revendications 2 à 4, **caractérisé en ce que** l'ester est le triacétate de glycérol.

6. Agent selon la revendication 1, **caractérisé en ce que** les radicaux R représentent des groupes -COOH.

7. Agent selon la revendication 6, **caractérisé en ce que** le composé répondant à la formule (I) représente l'acide citrique.

8. Agent selon l'une quelconque des revendications 6 ou 7, **caractérisé en ce que** l'ester se compose d'acide citrique et d'alcools contenant de 2 à 6 atomes de carbone.

9. Agent selon l'une quelconque des revendications 6 à 8, **caractérisé en ce que** l'ester est le citrate de triéthyle.

10. Agent selon l'une quelconque des revendications 1 à 9, **caractérisé en ce que** la phase huileuse est choisie parmi des huiles végétales, minérales et synthétiques, ainsi que des mélanges de ces composants.

11. Agent selon l'une quelconque des revendications 1 à 10, **caractérisé en ce qu'**il contient, à la place de la phase huileuse (B), un hydrocarbure halogéné liquide, à titre de troisième phase dans l'agent.

12. Agent selon la revendication 11, **caractérisé en ce que** les hydrocarbures halogénés liquides sont choisis parmi l'éther éthylperfluorobutylique et/ou le perfluorodécahydronaphtalène.

13. Agent selon l'une quelconque des revendications 1 à 12, **caractérisé en ce que** la phase (B) est présente en une quantité de 2 à 50 % en poids, rapportés au poids total de l'agent.

14. Agent selon l'une quelconque des revendications 1 à 13, **caractérisé en ce que** la phase aqueuse est présente en une quantité de 2 à 50 % en poids, rapportés au poids total de l'agent.

15. Agent selon l'une quelconque des revendications 1 à 14, **caractérisé en ce qu'**il contient en outre des agents filmogènes solubles dans l'eau.

16. Agent selon l'une quelconque des revendications 1 à 15, **caractérisé en ce qu'**il contient en outre des substances de soin solubles dans l'huile ou solubles dans l'eau, des absorbants du rayonnement ultraviolet, des huiles essentielles et/ou des colorants.

17. Agents selon l'une quelconque des revendications 1 à 16, **caractérisé en ce qu'**ils contiennent du panthénol.

18. Procédé pour donner du style à des cheveux, **caractérisé en ce qu'**on applique sur les cheveux un agent selon l'une quelconque des revendications 1 à 17, on met la chevelure en forme et on sèche ensuite les cheveux avec de l'air chaud ou simplement à l'air.

19. Utilisation d'un agent selon l'une quelconque des revendications 1 à 17 pour donner du style à des cheveux.

20. Utilisation d'un agent selon l'une quelconque des revendications 1 à 17 pour rendre des cheveux brillants.
